(19) Europäisches Patentamt European Patent Office Office européen des brevets

(11) EP 4 252 991 A1

(12) EUROPEAN PATENT APPLICATION
published in accordance with Art. 153(4) EPC

(43) Date of publication:
04.10.2023 Bulletin 2023/40

(21) Application number: 21915125.5

(22) Date of filing: 20.12.2021

(51) International Patent Classification (IPC):
B29B 17/02 (2006.01)        A61L 2/18 (2006.01)
A61L 101/10 (2006.01)

(52) Cooperative Patent Classification (CPC):
A61L 2/18; B29B 17/02; A61L 2101/00

(86) International application number:
PCT/JP2021/046931

(87) International publication number:
WO 2022/145265 (07.07.2022 Gazette 2022/27)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 28.12.2020 JP 2020219606

(71) Applicant: Unicharm Corporation
Shikokuchuo-shi, Ehime 799-0111 (JP)

(72) Inventors:
• KONISHI, Takayoshi
  Tokyo 108-8575 (JP)
• BANDOU, Takeshi
  Tokyo 108-8575 (JP)
• KURITA, Noritomo
  Tokyo 108-8575 (JP)

(74) Representative: Dolleymores
9 Rickmansworth Road
Watford, Hertfordshire WD18 0JU (GB)

(54) PREPROCESSING METHOD AND PREPROCESSING SYSTEM

(57) A pre-processing method that is executed as processing prior to a recycling process for recycling infectious waste into a material of new products, the pre-processing method according to the present invention includes a fragmenting step of fragmenting, as the infectious waste, a used non-woven fabric product mainly composed of a given thermoplastic resin; and a processing step of processing a part that is obtained at the fragmenting step using a given non-chlorine solution.

FIG.1

**Description**

Field

The present invention relates to a pre-processing method and a pre-processing system.

Background

In recent years, the worldwide demand for non-woven fabric products (for example, masks, diapers, etc.,) has been increasing. From the aspect of effective use of resources and reduction of waste, it is required to recycle and utilize non-woven fabric products.
A method of increasing efficiency of recycling in reusing and productizing used diapers and a method of performing a process of sterilizing infectious medical waste, reducing the volume of infectious medical waste, and making the infectious medical waste harmless and converting the processed waste into oil are known as an example of waste recycling.

Citation List

Patent Literature

    Patent Literature 1: Japanese Laid-open Patent Publication No. 2006-289154
    Patent Literature 2: International Publication Pamphlet No. WO 2014/357919

Summary

Technical Problem

The conventional technique, however, does not necessarily make it possible to exclude a risk of infection at a stage prior to a recycling process such that the infectious waste can be put into the recycling process for recycling into a material of new products.
For example, the above-described conventional technique only discloses a system on a site where the recycling process for recycling waste into a new product is performed and does not focus on exclusion of the risk of infection due to waste in a step of reaching to the site (for example, a transporting step).
For this reason, it is hard to say that the above-described conventional technique makes it possible to exclude the risk of infection at the stage prior to the recycling process such that infectious waste can be put into the recycling process for recycling into a material of new products.
The present application was made in view of the above-described circumstances and an object of the present application is to exclude the risk of infection at the stage prior to the recycling process such that infectious waste can be put into the recycling process for recycling into a material of new products.

Solution to Problem

According to an aspect of the present disclosure, a pre-processing method that is executed as processing prior to a recycling process for recycling infectious waste into a material of new products, the pre-processing method comprising: a fragmenting step of fragmenting, as the infectious waste, a used non-woven fabric product mainly composed of a given thermoplastic resin; and a processing step of processing a part that is obtained at the fragmenting step using a given non-chlorine solution. Advantageous Effects of Invention
According to a mode of an embodiment, it is possible to exclude a risk of infection at a stage prior to a recycling process such that infectious waste can be put into the recycling process for recycling into a material of new products.

Brief Description of Drawings

    FIG. 1 is a diagram illustrating a whole picture of a pre-processing method PT1 according to an embodiment;
    FIG. 2 is a sequence chart illustrating an example of the pre-processing method PT1 according to the embodiment;

FIG. 3 is a diagram illustrating a whole picture of a pre-processing method PT2 according to the embodiment;

FIG. 4 is a sequence chart illustrating an example of the pre-processing method PT2 according to the embodiment; and

FIG. 5 is a diagram illustrating an example of a blockchain according to the embodiment.

Description of Embodiments

The description and illustration of the present specification and the accompanying drawings demonstrate at least the following aspects.

A pre-processing method that is executed as processing prior to a recycling process for recycling infectious waste into a material of new products, the pre-processing method including: a fragmenting step of fragmenting, as the infectious waste, a used non-woven fabric product mainly composed of a given thermoplastic resin; and a processing step of processing a part that is obtained at the fragmenting step using a given non-chlorine solution.

According to the pre-processing method, because the used non-woven fabric product mainly composed of the given thermoplastic resin is fragmented and the part that is obtained by the fragmenting is processed with the given non-chlorine solution, it is possible to exclude a risk of infection at a stage prior to the recycling process such that the infectious waste can be put in the recycling process for recycling into the material of new products.

In the pre-processing method, hydrophobic non-woven fabric is separated as the used non-woven fabric product by fragmenting the non-woven fabric product whose non-woven fabric that is a constituent is hydrophobic and the processing step includes processing, with the given non-chlorine solution, the separated hydrophobic non-woven fabric as the part that is obtained at the fragmenting step.

According to the pre-processing method, because the hydrophobic non-woven fabric product whose non-woven fabric that is the constituent is hydrophobic is fragmented and accordingly the hydrophobic non-woven fabric is separated and the separated hydrophobic non-woven fabric is processed with the given non-chlorine solution, it is possible to perform the pre-processing appropriately on the hydrophobic non-woven fabric for chemical recycling.

In the pre-processing method, the processing step includes processing the part, which is obtained at the fragmenting step, using an oxidant non-chlorine solution as the given non-chlorine solution.

According to the pre-processing method, because the part that is obtained at the fragmenting step is processed using the oxidant non-chlorine solution, it is possible to obtain a recycling raw material that is accepted as a recycling object because the chlorine content is at or under a given value .

In the pre-processing method, the part that is obtained at the fragmenting step is further processed using a given polar solvent.

According to the pre-processing method, because the part that is obtained at the fragmenting step is further processed using the given polar solvent and accordingly it is possible to effectively separate high-polarity hydrophilic impurities (for example, hydrophilic stains and a hydrophilic material) from the low-polar hydrophobic part, for example, it is possible to obtain a recycling raw material with fewer impurities suitable for the chemical recycling.

A pre-processing method that is executed as processing prior to a recycling process for recycling infectious waste into a material of new products, the pre-processing method including: a fragmenting step of fragmenting, as the infectious waste, a non-woven fabric product that is a used non-woven fabric product that is mainly composed of a given thermoplastic resin and whose non-woven fabric that is a constituent is hydrophobic; and a processing step of processing hydrophobic non-woven fabric among parts that are obtained at the fragmenting step using a given polar solvent.

According to the pre-processing method, because the non-woven fabric product that is the used non-woven fabric product that is mainly composed of the given thermoplastic resin and whose non-woven fabric that is a constituent is hydrophobic is fragmented and the hydrophobic non-woven fabric among parts that are obtained by being fragmented is processed using the given polar solvent and accordingly it is possible to effectively separate high-polarity hydrophilic impurities (for example, hydrophilic stains and a hydrophilic material) from the low-polar hydrophobic non-woven fabric, for example, it is possible to obtain a recycling raw material with fewer impurities suitable for the chemical recycling.

In the pre-processing method, the hydrophobic non-woven fabric is non-woven fabric made of a hydrophobic thermoplastic resin.

According to the pre-processing method, because the hydrophobic non-woven fabric is non-woven fabric made of the hydrophobic thermoplastic resin, for example, it is possible to effectively obtain a polypropylene material or a polyethylene material as a recycling raw material.

In the pre-processing method, the fragmenting step includes fragmenting, as the given thermoplastic resin, a used non-woven fabric product mainly composed of a thermoplastic resin whose degree of crystallinity is at or under 75% or whose melting point is at or under 170 degree Celsius.

According to the pre-processing method, because the used non-woven fabric product mainly composed of the thermoplastic resin whose degree of crystallinity is at or under 75% or whose melting point is at or under 170 degree Celsius is fragmented, for example, it is possible to realize efficient conversion into oil in the chemical recycling.

In the pre-processing method, a non-woven fabric product containing polypropylene or polyethylene at a percentage of 95% or more is fragmented as the used non-woven fabric product mainly composed of the given thermoplastic resin.

According to the pre-processing method, because the non-woven fabric product containing polypropylene or polyethylene at the percentage of 95% or more (for example, at a percentage by weight of 95% or more) is fragmented as the used non-woven fabric product mainly composed of the given thermoplastic resin and accordingly, for example, it is possible to effectively obtain a polypropylene material or a polyethylene material as a recycling raw material, it is possible to reproduce a versatile plastic material by the chemical recycling.

In the pre-processing method, the part obtained at the fragmenting step is processed while being stirred together with the given disinfection solution in the gas phase.

According to the pre-processing method, because the part that is obtained at the fragmenting step is processed while being stirred together with the given disinfection solution (for example, a non-chlorine solution or a polar solvent) in the gas phase and accordingly it is possible to perform sterilizing and cleaning while applying physical damages to the part, it is possible to realize an increase in efficiency of sterilization, shortening of the time taken by a sterilizing process, and a reduction in the amount of the used disinfection solution.

In the processing method, a material that is determined as meeting the given condition among materials that are obtained via the processing step is extracted as a processed material on which the recycling process can be to be performed.

According to the processing method, because the material that is determined as meeting the given condition among the materials that are obtained via the processing step is extracted as a processed material on which the recycling process can be to be performed, it is possible to obtain a material in which certain quality or higher is maintained such that transportation to a recycling facility or conveyance to the next step can be performed.

In the pre-processing method, a material that is determined as meeting a condition that a chloride component that is contained is at or under a given value is extracted as the processed material among the materials obtained via the processing step.

According to the pre-processing method, because the material that is determined as meeting the condition that the chloride component that is contained is at or under the given value is extracted as the processed material among the materials obtained via the processing step and accordingly the environmentally conscious material that is accepted as substantially being free from chlorine can be subjected to recycling, it is possible to reproduce a substance that is safe. According to the eye processing method, it is possible to prevent generation of chlorine gas that is toxic and that has a facility corroding nature because of residual chlorine.

In the pre-processing method, a material that is determined as meeting a condition that an amount of residual bacteria is at or under a given value is extracted as the processed material among the materials obtained via the processing step.

According to the pre-processing method, because the material that is determined as meeting a condition that an amount of residual bacteria is at or under a given value is extracted as the processed material among the materials obtained via the processing step and accordingly it is possible to obtain a material in which the amount of bacteria because of, for example, colon bacteria, staphylococcal bacteria, and other bacteria is at or under a detection limit, it is possible to ensure safety of operating staffs and, as a result, it is possible to realize transportation to the recycling facility or conveyance to the next step.

In the pre-processing method, a material that is determined as meeting a condition that ash is at or under 3.0% is extracted as the processed material among the materials obtained via the processing step.

According to the pre-processing method, because the material that is determined as meeting a condition that ash is at or under 3.0% is extracted as the processed material among the materials obtained via the processing step, for example, in fiberization in material recycling, it is possible to efficiently obtain fibers having preferable fineness and a preferable length while realizing appropriate whiteness.

In the pre-processing method, the non-woven fabric product is crushed under a given environment that makes it possible to prevent bacteria from scattering as a process of fragmenting the used non-woven fabric product mainly composed of the given thermoplastic resin and a part after crushing is processed.

According to the pre-processing method, because the non-woven fabric product is crushed under the given environment that makes it possible to prevent bacteria from scattering as the process of fragmenting the used non-woven fabric product mainly composed of the given thermoplastic resin and a part after crushing is processed, it is possible to inhibit the risk of scattering of bacteria at each step contained in the pre-processing.

In the pre-processing method, the used non-woven fabric product is crushed under an underwater environment or an exhaust environment.

According to the pre-processing method, because the used non-woven fabric product is crushed under the underwater environment or the exhaust environment, it is possible to inhibit a risk of scattering of bacteria at each step contained in the pre-processing.

In the pre-processing method, the used non-woven fabric product is crushed such that a size after crushing is within a given range.

According to the pre-processing method, because the used non-woven fabric product is crushed such that the size after crushing is within the given range, it is possible to obtain a recycling raw material that can meet both a condition of a size optimum to thermal processing and a condition of a size optimum to inhibition of the risk of scattering.

An example of a mode for carrying out the pre-processing method (referred to as "embodiment" below) will be described in detail below with reference to the drawings. Note that the embodiment does not limit the pre-processing method and a pre-processing system. In the following embodiment, the same parts are denoted with the same reference numerals and redundant description will be omitted.

Embodiment

1. Overview of Pre-processing Method according to Embodiment

First of all, an overview of a pre-processing method according to an embodiment will be described. For example, from the aspect of effective use of resources and reduction of waste, it is required to recycle and utilize used non-woven fabric products. Used masks and used diapers are exemplified as representatives of used non-woven fabric products; however, the products have to be dealt with as infectious waste because they are used with their direct contact with human skins.

In a specific example, when such infectious used non-woven fabric products as those described above are to be recycled, before the non-woven fabric products are caused to reach a recycling facility that is a site where a central process (for example, material recycling or chemical recycling) of recycling into a material of new products is performed, a specific step in which scattering of bacteria is concerned occurs.

For example, when non-woven fabric products that are collected are to be recycled, a material that is put into a state more suitable for recycling by separating and fragmenting, crushing, cleaning, etc., is processed as a subject to be recycled in a recycling factory. A step of separating and fragmenting crushing, cleaning, etc., is a step that is taken as pre-processing for recycling and a pre-processing facility where the pre-processing step is taken and a recycling facility that is a facility where actual recycling is performed are often separated.

For example, the pre-processing facility and the recycling facility may be on the same site or may be in areas different from each other (for example, across prefectures) and, in any case, a transporting step of transporting objects to be recycled from the pre-processing facility to the recycling facility occurs. Not limited to the concerned example, when there is a shift to the next step, for example, from the specific step (for example, a fragmenting and separating step) to another step (for example, a sterilizing step), a conveying step of conveying a material may occur.

In the transporting step or the conveying step, there is a possibility that non-woven fabric products go into an open air space and thus there is a concern about scattering of bacteria. Thus, there is a problem in that transportation to the recycling facility or conveyance to the next step cannot be performed without making an adjustment on the used non-woven fabric products to keep a certain quality or more at the time of the pre-processing that is a stage prior to putting of the objects to be recycled into the recycling facility.

The certain quality or more herein indicates that various types of conditions that are determined for a processing subject on which the recycling process is to be performed, such as, starting with being harmless, chlorine content at a given value or smaller, an amount of residual bacteria at a given value or smaller, and ash at a given value or smaller, are met at the stage of the pre-processing. It is not possible to move to the recycling process unless the quality is not kept by meeting the conditions.

Thus, the pre-processing method according to the embodiment is an approach for solving such a problem as that described above and is a pre-processing method that is executed as the pre-processing that is performed at the stage prior to the recycling process for recycling infectious waste into a material of new products. Specifically, the pre-processing method according to the embodiment is a method of preparing for a subject to be recycled in which the certain quality or more is ensured by meeting the recycling conditions while excluding the risk of infection that can occur in the transportation step or a shift to the next process.

In the pre-processing method according to the embodiment, a sterilizing and cleaning method unique to a combination of characteristics of non-woven fabric products and the type of recycling is employed according to characteristics of used non-woven fabric products and the type of recycling. For example, in the pre-processing method according to the embodiment, when material recycling or chemical recycling is performed on used non-woven fabric products having a characteristic of being composed mainly of thermoplastic resin, a method of processing with a non-chlorine solution is employed. On the other hand, for example, in the pre-processing method according to the embodiment, when chemical recycling is performed on used non-woven fabric products having a characteristic that non-woven fabric members of the constituents are hydrophobic, a method of processing using a given polar solvent is employed.

2. About Non-woven Fabric Product

Non-woven fabric products on which the pre-processing method is to be performed according to the embodiment will be described next. The non-woven fabric products on which the pre-processing method is to be performed according to the embodiment include products mainly made of non-woven fabric, such as non-woven masks, non-woven gowns, non-woven caps, wipes, and cleaning sheets, and absorbent products, such as disposable diapers, pads, sanitary napkins, pads, bed sheets, and pet sheets.

When absorbent items are processed by the pre-processing method according to the embodiment, it is necessary to fragment the absorbent items or stir them in a solvent and, after separating the absorbent and non-woven sheets, separate the absorbent sheets from other parts (for example, pulp, absorbent polymer, plastic sheets).

Hydrophobic non-woven fabric will be described, too. Hydrophobic non-woven fabric is non-woven fabric made of hydrophobic thermoplastic resin. For example, polyethylene, polyolefin, such as polypropylene, polyester, such as polyethylene terephthalate, etc., are taken as hydrophobic thermoplastic resin. In other words, hydrophobic non-woven fabric denotes any one of these hydrophobic thermoplastic resins or non-woven fabric into which multiple hydrophobic thermoplastic resins are fiberized.

Furthermore, spunbond non-woven fabric, air-through non-woven fabric, melt-blown non-woven fabric, SMS non-woven fabric obtained by layering sheets of these fabrics, furthermore, point-bond non-woven fabric, needle-punched non-woven fabric, spun-lace non-woven fabric, etc., are taken as an example of other hydrophobic non-woven fabric.

3. Pre-processing Method according to Embodiment

The pre-processing method according to the embodiment can be divided into a versatile pre-processing method (referred to as "pre-processing method PT1") that is applicable regardless of the type of recycling (specifically, material recycling or chemical recycling) as described above and a pre-processing method (pre-processing method PT2) that is particularly effective in a specific type of recycling (specifically, chemical recycling).

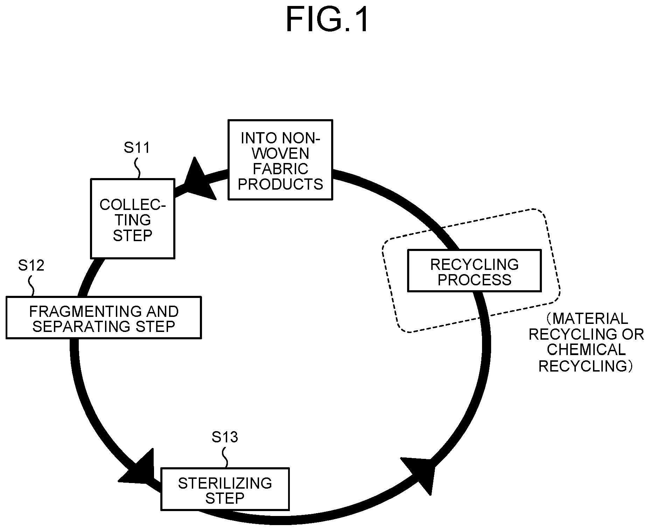

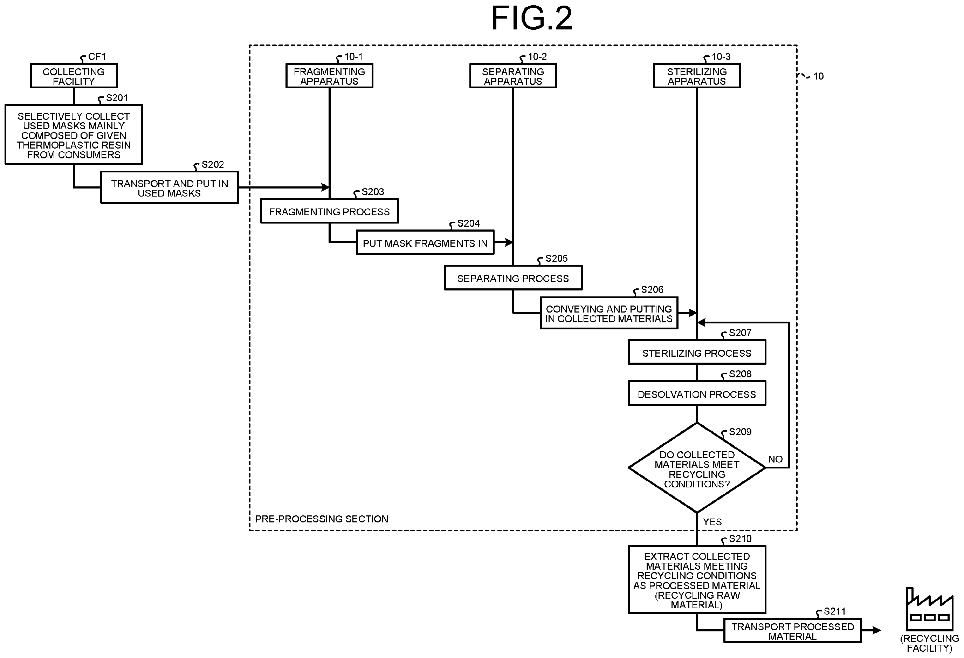

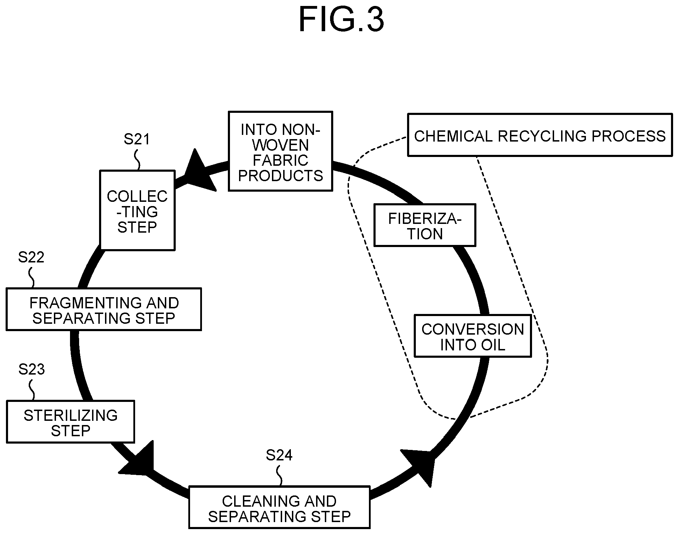

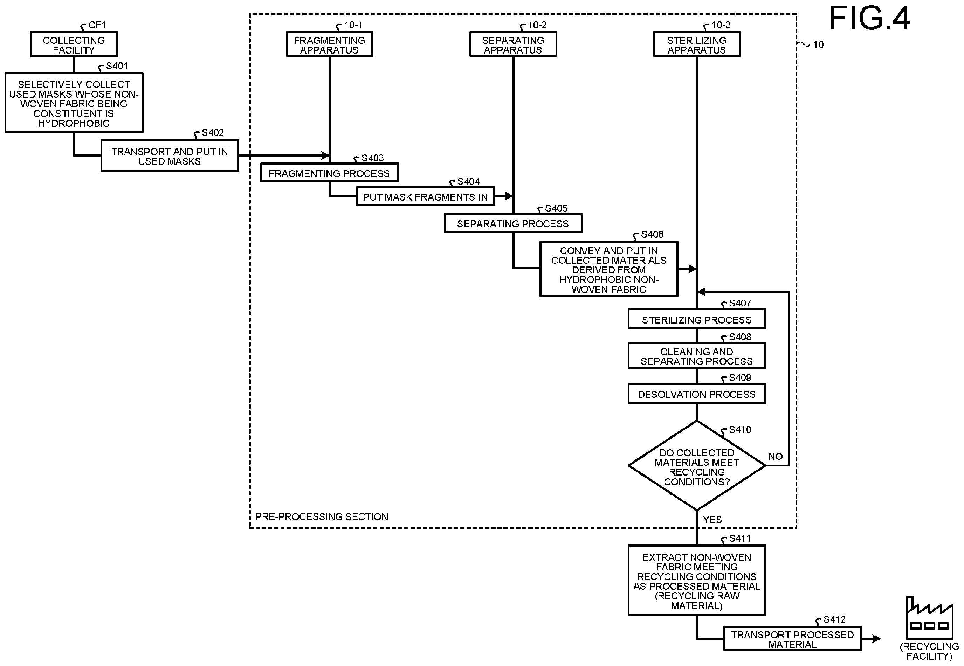

Thus, in the following description, the "pre-processing method PT1" is focused in FIG. 1 and FIG. 2 and the "pre-processing method PT1" is focused in FIG. 3 and FIG. 4 and each of the methods will be described.

3-1. Whole image of Pre-processing method PT1

Using FIG. 1, an overall flow of the pre-processing method PT1 according to the embodiment will be described. FIG. 1 is a diagram illustrating a whole picture of the pre-processing method PT1 according to the embodiment. FIG. 1 conceptually illustrates a cycle in which used non-woven fabric products that are used by consumers are collected, the pre-processing is performed on the collected used non-woven fabric products, and the recycling process (the material recycling or the chemical recycling) is performed on the pre-processed non-woven fabric products so that the non-woven fabric products change into new non-woven fabric products.

FIG. 1 illustrates main steps of steps contained in the pre-processing method PT1 according to the procedure. According to the example in FIG. 1, the pre-processing method PT1 is a method consisting of a plurality of steps that are a collecting step, the fragmenting and separating step, and the sterilizing step. As illustrated in FIG. 1, the pre-processing method PT1 is realized in the pre-processing system that performs the collecting step at step S11, performs the fragmenting and separating step at step S12, and the sterilizing step at step S13. The recycling process is performed actually on a material that is obtained through these steps.

About Collecting Step

The collecting step at S11 will be described. In the collecting step, used non-woven fabric products mainly composed of given thermoplastic resin are selectively collected from consumers. The collecting step may employ a collecting method in which a collecting box in which used non-woven fabric products mainly composed of the given thermoplastic resin can be put is set in any collecting facility (for example, a supermarket, a hospital or a public facility of a local government, or the like) and they are selectively collected.

Assume here, for example, that products that are made by a manufacturer M1 and that belong to a brand B1 include a mask product PD1 and the mask product PD1 is a product mainly composed of the given thermoplastic resin. Thus, for example, to selectively collect the mask products PD1 that are used from consumers, the collecting step may employ a collecting method in which a collecting box having a system in which the mask products PD1 are put into the collecting box appropriately is set in a collecting facility.

When the due date on which the collecting box is transported to the pre-processing facility (for example, a plant for the pre-processing) comes or the collecting box is full, next move to the fragmenting and separating step at step S12. For example, the collecting box that is obtained at the collecting step is transported in a sealed manner to the pre-processing facility and, at the pre-processing facility to which the collecting box is transported, the fragmenting process and the separating process on the used non-woven fabric products in the collecting box are performed.

About Fragmenting and Separating Step

The fragmenting and separating step at step S12 will be described. At the fragmenting and separating step, the fragmenting process of fragmenting used non-woven fabric products mainly composed of the given thermoplastic resin and the separating process of separating and collecting constituents mainly composed of the given thermoplastic resin among the constituents composing the non-woven fabric products are performed.

For example, at the fragmenting and separating step, the used non-woven fabric products are fragmented. For example, in the fragmenting and separating step, the used non-woven fabric products are crushed into a given size and as a result are fragmented into sheet fragments (for example, sheet-like non-woven fabric fragments).

For example, a used non-woven fabric product sometimes consists of various parts, such as non-woven fabric, various types of plastic parts (for example, films), rubber parts, absorbent materials (for example, SAP; Superabsorbent polymer), cellulose fibers, metal parts, and adhesives. Thus, in the next separating process, a separating process of separating and collecting fragments that are mainly non-woven fabric that is a constituent mainly composed of the given thermoplastic resin is performed. In general, the non-woven fabric has a form of sheet and thus non-woven fabric fragments that are fragments that are mainly non-woven fabric can be referred to as sheet fragments.

Thus, in the separating process, in addition to separation of sheet fragments that are fragments that are mainly non-woven fabric from the fragments including various constituents, such as non-woven fabric, various types of plastic parts (for example, films), rubber parts, absorbent materials (for example, SAP), cellulose fiber, metal parts, and adhesives, the sheet fragments are separated also from various foreign materials.

In the fragmenting and separating step, it is preferable to fragment non-woven fabric products composed of only polypropylene and polyethylene materials.

Sterilizing Step

A sterilizing step at step S13 will be described. For example, to be accepted as a recycling object on which the recycling process is to be performed, it is necessary to meet the recycling conditions like those illustrated in FIG. 1. Specifically, a material that meets the recycling conditions that "the chlorine content is at or under N11%", "the amount of residual bacteria is at N12%", and "ash is at or under N13%" is accepted as a recycling object that is an object on which the recycling process is to be performed and is subjected to the recycling process.

For this reason, at the sterilizing step, the sterilizing process of performing sterilizing (disinfection) cleaning on the collected materials that are separated and collected is performed to obtain a material that meets the above-described recycling conditions. For example, at the sterilizing step, the sterilizing process of performing sterilizing and cleaning on the collected materials that are separated and collected using a given non-chlorine solution is performed. An ozone solution (for example, ozone water) is taken as the non-chlorine solution.

Summary

As described above, in the pre-processing method PT1 that is executed as the pre-processing of the recycling process, used non-woven fabric products mainly composed of the given thermoplastic resin are selectively collected (the collecting step), the collected used non-woven fabric products are fragmented and accordingly the constituents (for example, non-woven fabric) mainly composed of the given thermoplastic resin are separated and collected (the fragmenting and separating step), and the separated and collected constituents are sterilized and cleaned using a given non-chlorine solution (the sterilizing step).

As a result of processing of the used non-woven fabric products that are mainly composed of the given thermoplastic resin by the pre-processing method PT1, as illustrated in FIG. 1, the recycling conditions that "the chlorine content is at or under N11%", "the amount of residual bacteria is at or under N12%", and "ash is at or under N13%" are met so that a material that can be substantially accepted as being made harmless is obtained. Thus, the material is determined as "a processed material" that has been processed such that the material can be subjected to the recycling process and is transported to the recycling facility where the recycling process is performed.

The processed material meets the recycling conditions and thus can be referred to as a material in which certain quality or higher is maintained and obtaining the material at the stage prior to the recycling process by the pre-processing method PT1 makes it possible to effectively inhibit scattering of bacteria in transportation to the recycling facility and conveyance to the next step. In other words, according to the pre-processing method PT1, it is possible to exclude the risk of infection at the stage prior to the recycling process.

For the conceptually-presented values like the chlorine content of N11%, the amount of residual bacteria of N12%", and ash of N13%, optimum real numbers are set in accordance with the situation on the processing site. Specific examples of the recycling conditions are presented below.

For example, as for the specific example of the chlorine content of N11%, setting the concentration of chlorine gas that occurs at or under 1 ppm when the material that is processed by the pre-processing method PT1 is heated to a melting point or higher and is gasified is taken as an example. The value of the concentration of 1 ppm is a value achievable by selecting what to be collected and processing with the non-chlorine solution.

As for the specific example of the amount of residual bacteria of N12%, being lower than a value that is determined by linen standards, being at or under a detection limit as for the amount of residual bacteria of a colon bacterial group and staphylococcus aureus (MRSA), and 10,000 bacteria or less per 100 $cm^2$ as for the amount of remaining other bacteria are taken as an example.

As for the specific example of ash of N13%, ash under 10% (more preferably, under 7% and, more preferably, under 3%) is taken as an example.

Note that the three items of the chlorine content at or under N11%, the amount of residual bacteria at or under N12%, and ash at or under N13% are exemplified; however, the three items need not necessarily be satisfied.

The chlorine content has an effect on apparatuses and devices that are used in the pre-processing step and the recycling step and chemical reactions, the amount of residual bacteria has an effect on safety of operating staffs and on whether acceptance is enabled according to the level of measures against bacteria at various types of process steps and therefore they are thought to have an effect on versatility. Ash has an effect on the properties of the material after recycling and therefore an appropriate value has to be achieved according to the purpose at the sage of the pre-processing step.

3-2. Specific Example of Pre-processing Method PT1

Using FIG. 2, a specific example of the pre-processing method PT1 according to the embodiment will be described. FIG. 2 is a sequence chart illustrating an example of the pre-processing method PT1 according to the embodiment. FIG. 2 illustrates a detailed example of the pre-processing method PT1 described using FIG. 1. FIG. 2 illustrates an example in which the pre-processing method PT1 is performed among a collecting facility CF1, a fragmenting apparatus 10-1, a separating apparatus 10-2, and a sterilizing apparatus 10-3.

In the collecting facility CF1, product collection corresponding to the collecting step among the process steps constituting the pre-processing method PT1 is performed. The fragmenting apparatus 10-1 performs the fragmenting process contained in the fragmenting and separating step among the process steps constituting the pre-processing method PT1. The separating apparatus 10-2 performs the separating process contained in the fragmenting and separating step among the process steps constituting the pre-processing method PT1. The sterilizing apparatus 10-3 performs the sterilizing process corresponding to the sterilizing step among the process steps constituting the pre-processing method PT1.

In the example in FIG. 2, the fragmenting apparatus 10-1, the separating apparatus 10-2, and the sterilizing apparatus 10-3 are contained in a pre-processing section 10. The pre-processing section 10 is a section for used non-woven fabric products collected in the collecting facility CF1 to undergo the pre-processing for the recycling process and corresponds to, for example, a single pre-processing facility (a single plant for the pre-processing). The pre-processing section 10 may be divided into multiple sections. For example, the pre-processing section 10 may be divided into multiple sections (multiple plants for the pre-processing) in accordance with apparatuses that are contained.

In the example in FIG. 2, non-woven fabric products to be processed by the pre-processing method PT1 will be described as masks (mask products) below. Assume that the mask products are products manufactured by the manufacturer M1 and are the mask products PD1 that belong to the brand B1.

The mask product PD1 is a mask mainly composed of the given thermoplastic resin. In this respect, for example, the mask product PD1 may be a mask mainly composed of a thermoplastic resin whose degree of crystallinity is at or under 75% or whose melting point is at or under 170 degrees. For example, the mask product PD1 may be a mask containing polypropylene or polyethylene at a percentage of 95% or more (for example, at a percentage by weight of 95% or more).

The main constituent constituting the mask product PD1 is non-woven fabric. Thus, the mask product PD1 may be a mask containing hydrophobic non-woven fabric as a constituent. In other words, the mask product PD1 may be a mask containing, as a constituent, non-woven fabric that is mainly composed of a hydrophobic thermoplastic resin and thus presents hydrophobicity. In this respect, for example, the mask product PD1 may be a mask that contains, as a constituent, non-woven fabric mainly composed of a thermoplastic resin whose degree of crystallinity is at or under 75% or whose melting point is at or under 170 degree Celsius and that thus represents hydrophobicity. The mask product PD1 may be a mask that contains, as a constituent, non-woven fabric that contains polypropylene or polyethylene at a percentage of 95% or more (for example, at a percentage by weight of 95% or more) and that thus represents hydrophobicity.

In the state, in the example in FIG. 2, first of all, the collecting facility CF1 that is a supermarket collects used masks mainly composed of the given thermoplastic resin, that is, used mask products PD1 selectively from consumers (step S201). The collecting facility CF1 is able to employ a collecting method of various systems such that the used mask products PD1 can be collected selectively.

An example of the collecting method that can be employed will be presented. For example, the collecting facility CF1 is able to employ a collecting method in which a collecting box with a system by which the mask products PD1 are put in the collecting box appropriately is set in the store.

A method in which information enabling consumers to recognize that masks that can be put into are the mask products PD1 (for example, a brand name, a product name, an item number, and a package image corresponding to the mask product PD1, an image of the body of the mask product PD1, etc.,) is specified in appropriate part (for example, a lid part) is considered an example of the system by which the mask products PD1 are put in. Specific examples of masks that cannot be put in (for example, masks containing silver components, fabric masks, and masks made of vinyl chloride, etc.,) on the collecting box is effective, too.

Setting a collecting box having a sorting function is another example of the system by which the mask products PD1 are put in appropriately. For example, as described above, only specifying that masks that can be put in are the mask products PD1 as described above does not necessarily enable only the used mask products PD1 to be put in. For this reason, when the collecting box has the function enabling sorting of the mask products PD1, it is possible to collect the mask products PD1 more selectively.

For example, on sensing that a mask is put in, the collecting box determines whether the mask that is put in is the mask product PD1 by image determination using a freely-selected image analysis technique. On determining that the mask that is put in is not the mask product PD1, the collecting box may make a warning, such as an alert, to a person who puts the mask in to make the person to take the mask out. On determining that the mask that is put in is not the mask product PD1, the collecting box may eject the mask that is put in from a given ejection port by an automatic sorting function. On determining that the mask that is put in is not the mask product PD1, the collecting box may crush the mask that is put in with a shredder.

The collecting box may determine whether the mask that is put in is the mask product PD1 not based on image determination but based on, for example, reading of identification information that is added to the mask. Assume, for example, that the mask product PD1 is tagged with given identification information (for example, a chip or a code) and the collecting box has the tagged identification information as correct data. In that case, on sensing that some mask is put in, the collecting box determines whether the mask that is put in is the mask product PD1 by making analyses on whether the sensed mask is added with identification information in the first place and, when added, on whether the identification information matches the correct data.

Making a putting-in assist using a digital technique such that a consumer is able to put the mask product PD1 in the collecting box appropriately is another example of the system by which the mask products PD1 are put in appropriately. According to the digital technique, when putting it in the collecting box, a consumer acquires a dedicated QR code (trademark) using a terminal device (for example, a smartphone, or the like) of the consumer. The consumer then causes the collecting box to read the acquired QR code. On recognizing that the QR code is appropriate, the collecting box unlocks the lid, opens automatically, and induces putting of the mask product PD1 in the collecting box. For example, the collecting box may output information for checking that the mask to be put in is the mask product PD1 on a screen that the collecting box includes. A system in which information for checking that the mask to be put in is the mask product PD1 on the terminal device of the consumer may be employed.

The used mask products PD1 that are collected in the colleting facility CF1 from consumers, for example, are transported by a given carrier to the pre-processing section 10 (step S202). For example, when a due date that is determined in the collecting facility CF1 comes, or when the collecting box that is set is full, the used mask products PD1 are transported by the given carrier to the pre-processing section 10. For example, the used mask products PD1 are transported in a sealed state in the collecting box to the pre-processing section 10.

When the used mask products PD1 arrive, an operating staff in the pre-processing section 10 works to put the used mask products PD1 in the fragmenting apparatus 10-1 while reducing the risk of infection and ensuring safety (for example, prevention of scattering and sterilization of droplets).

The fragmenting apparatus 10-1 performs the fragmenting process of fragmenting the used mask products PD1 finely by crushing (step S203). For example, the fragmenting apparatus 10-1 may crush the used mask products PD1 in an underwater environment or an exhaust environment. For example, the fragmenting apparatus 10-1 crushes the used mask products PD1 while performing sterilization in a given disinfection solution environment or a given exhaust environment. For example, the fragmenting apparatus 10-1 has a tank and the used mask products PD1 are put in the tank with a given disinfection solution therein.

The given disinfection solution may be, for example, an aqueous solution into which a disinfection agent is mixed. Ozone, chlorine dioxide, hypochlorous acid, a hydrogen peroxide solution, peracetic acid, or the like, is taken as the disinfection agent. When water is used simply as the given disinfection solution, it is preferable to set water at a temperature (for example, at or above 70 degree Celsius) that is expected to increase the sterilizing effect.

In the state, the fragmenting apparatus 10-1 pours the used mask products PD1 together with the given disinfection solution to a coaxial crusher from a port with which the tank is provided and accordingly crushes the used mask products PD1 while performing sterilization. Accordingly, the fragmenting apparatus 10-1 is able to fragment the used mask products PD1 while effectively inhibiting an increase in the risk of infection caused by scattering of bacteria from the used mask products PD1. The fragmenting apparatus 10-1 may perform crushing further in combination with, for example, heat sterilization using high-temperature steam, hot water, hot air, or heated oil or UV sterilization by UV irradiation.

At step S203, the fragmenting apparatus 10-1 fragments the used mask products PD1 such that the size after fragmentation is within a given range. For example, the fragmenting apparatus 10-1 crushes the used masks PD1 by operating the coaxial crusher that is adjusted such that the size of a mask fragment obtained by crushing the used mask products PD1 is within a range of 1 to 50 mm.

For example, when the size after fragmentation is too large, there is a risk that heat does not diffuse uniformly throughout the material in thermal processing that is performed in the chemical recycling or sterilization of the material cannot be performed sufficiently. A too small size after fragmentation results in fine powders and there is a risk of an increase in the risk of scattering into the air (that is, a risk of scattering of bacteria). Thus, a range of 1 to 50 mm is set as a size condition that can meet both a condition of a size optimum to the thermal processing and a condition of a size optimum to inhibition of the risk of scattering.

The mask fragments that are fragments obtained by the fragmenting process are put in the separating apparatus 10-2 (step S204). For example, the operating staff of the pre-processing section 10 may put the mask fragments in while reducing the risk of infection and ensuring safety or a mechanism in which the mask fragments are automatically put in the separating apparatus 10-2 from the fragmenting apparatus 10-1 may be employed between the fragmenting apparatus 10-1 and the separating apparatus 10-2. For example, when the separating apparatus 10-2 is under the fragmenting apparatus 10-1, the mask fragments are put in the separating apparatus 10-2 from the fragmenting apparatus 10-1.

Once the mask fragments are put in, the separating apparatus 10-2 performs the separating process of separating and collecting, from the mask fragments that are put in, mask fragments (that is, sheet fragments) that are mainly the non-woven fabric that is the constituent mainly composed of the given thermoplastic resin (step S205). For example, the mask product PD1 consists of various parts, such as non-woven fabric, various types of plastic parts (for example, films), rubber parts, absorbent materials (for example, SAP), cellulose fibers, metal parts, and adhesives. Thus, the separating apparatus 10-2 separates sheet fragments (non-woven fabric fragments) that are the mask fragments that are mainly the non-woven fabric from the mask fragments containing these various constituents. The separating apparatus 10-2 separates sheet fragments from other various foreign materials.

For example, the separating apparatus 10-2 may be a harpe separator having a cleaning tank and a sifting tank. According to the separating apparatus 10-2, the mask fragments are separated into sheet fragments (non-woven fragments) and other substances (for example, mask fragments that are mainly constituents other than the non-woven fabric and other foreign materials) while being cleaned. The sheet fragments do not pass through the screen and thus result in remaining in the separating apparatus 10-2 and other substances are ejected. Accordingly, when the mask fragments are put in, the separating apparatus 10-2 collects sheet fragments that are mainly the non-woven fabric from the mask fragments that are put in.

As described above, one that can be a recycling raw material among the constituents composing the mask product PD1 is sometimes not limited to non-woven fabric. For example, there is a possibility that various types of plastic members, such as films, and thermoplastic resin for rubber members are main components. For this reason, the separating apparatus 10-2 may separate and collect mask fragments that are mainly films and rubber members from the mask fragments that are put in.

The collected materials that are the sheet fragments that are separated and collected by the separating apparatus 10-2 are conveyed to the sterilizing apparatus 10-3 (step S206). For example, the operating staff in the pre-processing section 10 conveys the collected materials to the sterilizing apparatus 10-3 and puts the conveyed collected material in the sterilizing apparatus 10-3. The operating staff works while reducing the risk of infection and ensuring safety (for example, prevention of scattering and sterilization of droplets).

Once the collected materials are put in, the sterilizing apparatus 10-3 performs the sterilizing process of sterilizing and cleaning the collected materials such that materials that meet the recycling conditions are obtained (step S207). For example, the sterilizing apparatus 10-3 sterilizes and cleans the collected materials using the given non-chlorine solution. For example, the sterilizing apparatus 10-3 sterilizes and cleans the collected materials using an oxidant non-chlorine solution (for example, an ozone solution, such as ozone water) as the given non-chlorine solution.

For example, the sterilizing apparatus 10-3 may sterilize and clean the collected materials by spraying the given non-chlorine solution while stirring the collected materials in a gas phase. The sterilizing process sterilizes and cleans the collected materials while applying physical damages to the collected materials and therefore makes it possible to realize an increase in efficiency of sterilization, shortening of the time taken by the sterilizing process and a reduction in the amount of the used solution.

The sterilizing apparatus 10-3 will be described more in detail. The sterilizing apparatus 10-3 has an overturned cylindrical part provide with a plurality of blades inside. An upper part (ceiling) of the cylindrical part is provided with a mechanism for applying a disinfection solution to the collected materials that are put in the cylindrical part and a lower part (bottom) of the cylindrical part is provided with a mechanism for transmitting and removing fine foreign matters.

Accordingly, with the blades being rotated, the sterilizing apparatus 10-3 sterilizes and cleans the collected materials that are put in the cylindrical part while applying the given non-chlorine solution to the collected materials. More specifically, while rotating the blades to send the collected materials from the port of the cylindrical part to the exit, the sterilizing apparatus 10-3 performs the sterilizing process of applying the given non-chlorine solution to the collected materials. According to the sterilizing process, the collected materials are sterilized and cleaned with the given non-chlorine solution while being applied with physical damages, which makes it possible to realize effective sterilization (disinfection) and removal of stains and foreign matters. The cleaning process makes, as a result, the collected materials meet the recycling conditions.

When the sterilizing process at step S207 ends, the sterilizing apparatus 10-3 performs a desolvation process on the collected materials on which the sterilizing process has been performed, thereby making an adjustment such that the moisture content of the collected materials is at or under a given value (step S208).

The sterilizing apparatus 10-3 performs an evaluating process of evaluating whether the collected materials after desolvation meet the recycling conditions (step S209). Specifically, the sterilizing apparatus 10-3 evaluates whether the collected materials after desolvation meet the recycling conditions (the conditions for being accepted as recycling objects that are objects to be recycled) that "the chlorine content is at or under N11%", "the amount of residual bacteria is at or under N12%", and "ash is at or under N13% (for example, 3.0%)". A freely-selected conventional method may be employed for evaluation. For example, the sterilizing apparatus 10-3 is able to collect part of the collected materials after desolvation as a sample and perform the evaluating process on the collected sample.

When an evaluation result presenting that any one of the recycling conditions is not met is obtained (NO at step S209), the process from step S207 is repeated until an evaluation result presenting that all the recycling conditions are met is obtained.

When the evaluation result presenting that all the recycling conditions are met is obtained (YES at step S209), the sterilizing apparatus 10-3 moves to an extracting step. For example, when the evaluation result presenting that all the recycling conditions are met is obtained, the sterilizing apparatus 10-3 extracts the collected materials that meet all the recycling conditions as processed materials (the recycling raw material) in which certain quality or higher is ensured such that the materials can be subjected to the recycling process (step S210).

Note that, at step S209, all the recycling conditions need not necessarily be met and, for example, recycling conditions that should be met may be changed according to the purpose of recycling. The process of steps S208 to S210 may be performed by an apparatus other than the sterilizing apparatus 10-3. Particularly, the evaluating process at step S209 may be performed manually by the operating staff.

The collected processed materials are transported to a given recycling facility according to the purpose (whether it is material recycling or chemical recycling) (step S211).

The specific example of the pre-processing method PT1 according to the embodiment has been described using FIG. 2. According to the pre-processing method PT1 described above, it is possible to obtain the effects below in addition to the effect that it is possible to exclude the risk of infection at the stage prior to the recycling process. Specifically, according to the pre-processing method PT1 according to the embodiment, because mixture of foreign matters deriving from inorganic matters is inhibited (specifically, because the ash is reduced at or under 3.0%), in fiberization for obtaining non-woven fiber products, such as masks and diapers, by recycling, it is possible to efficiently obtain fibers having preferable fineness and a preferable length while realizing appropriate whiteness. According to the pre-processing method PT1, it is possible to realize an increase in a yield of collected components after conversion into oil. According to the pre-processing method PT1, because the recycling process using a chlorine-free material is performed, it is possible to reproduce products that is gentle on skin, secure, and safe.

3-3. Whole Picture of Pre-processing Method PT2

Using FIG. 3, an overall flow of the pre-processing method PT2 according to the embodiment will be described. FIG. 3 is a diagram illustrating a whole picture of the pre-processing method PT2 according to the embodiment. FIG. 3 conceptually illustrates a cycle in which used non-woven fabric products that are used by consumers are collected, pre-processing is performed on the collected used non-woven fabric products, and a recycling process (chemical recycling) is performed on the pre-processed non-woven fabric products so that the non-woven fabric products change into new non-woven fabric products.

It has been described that the pre-processing method PT1 described above is a versatile method that is applicable in any of the case where the recycling process to be performed later is material recycling and the case where the recycling process is chemical recycling. On the other hand, the pre-processing method PT2 is different from the pre-processing method PT1 in processing using a given polar solvent on hydrophobic non-woven fabric among constituents composing a non-woven fabric product such that an effect is exerted particularly on chemical recycling.

Note that many techniques employed for the pre-processing method PT2 are common to or similar to the pre-processing method PT1. Thus, aspects that are common to or similar to those according to the description of FIG. 1 in the description of FIG. 3 will be omitted as appropriate.

FIG. 3 illustrates main steps of steps contained in the pre-processing method PT2 according to the procedure. According to the example in FIG. 3, the pre-processing method PT1 is a method consisting of a plurality of steps that are a collecting step, a fragmenting and separating step, a sterilizing step, and a cleaning and separating step. As illustrated in FIG. 3, the pre-processing method PT2 is realized in the pre-processing system that performs the collecting step at step S21, performs the fragmenting and separating step at step S22, the sterilizing step at step S23, and the cleaning and separating step at step S24. The recycling process is performed actually on a material that is obtained through these steps.

About Collecting Step

The collecting step at S21 will be described. In the collecting step, used non-woven fabric products mainly composed of given thermoplastic resin are selectively collected from consumers. More specifically, in the collecting step, non-woven fabric products that are used non-woven fabric products mainly composed of a given thermoplastic resin and whose non-woven fabric that is a constituent is hydrophobic are selectively collected from consumers. The collecting step may employ a collecting method in which a collecting box in which non-woven fabric products that are used non-woven fabric products mainly composed of the given thermoplastic resin and whose non-woven fabric that is a constituent is hydrophobic can be put is set in any collecting facility (for example, a supermarket, a hospital or a public facility of a local government, or the like) and they are selectively collected.

Assume here, for example, that products that are made by a manufacturer M1 and that belong to a brand B1 include a mask product PD2 and the mask product PD2 is a product mainly composed of the given thermoplastic resin and non-woven fabric that is a constituent is hydrophobic. Thus, for example, to selectively collect the mask products PD2 that are used from consumers, the collecting step may employ a collecting method in which a collecting box having a system in which the mask products PD2 are put into the collecting box appropriately is set in a collecting facility.

When the due date on which the collecting box is transported to the pre-processing facility (for example, a plant for the pre-processing) comes or the collecting box is full, next move to the fragmenting step at step S22. For example, the collecting box that is obtained at the collecting step is transported in a sealed manner to the pre-processing facility and, at the pre-processing facility to which the collecting box is transported, the fragmenting process and the separating process on the used non-woven fabric products in the collecting box are performed.

About Fragmenting and Separating Step

The fragmenting and separating step at step S22 will be described. At the fragmenting and separating step, the fragmenting process of fragmenting used non-woven fabric products mainly composed of the given thermoplastic resin and whose non-woven fabric that is a constituent is hydrophobic and the separating process of separating and collecting the hydrophobic non-woven fabric among the constituents composing the non-woven fabric products are performed.

For example, at the fragmenting and separating step, the used non-woven fabric products are fragmented. For example, in the fragmenting and separating step, the used non-woven fabric products are crushed into a given size and as a result are fragmented into sheet fragments (for example, sheet-like non-woven fabric fragments).

For example, a used non-woven fabric product sometimes consists of various parts, such as non-woven fabric, various types of plastic parts (for example, films), rubber, absorbent materials (for example, SAP), cellulose fibers, metal parts, and adhesives. Thus, in the next separating process, a separating process of separating and collecting fragments that are non-woven fabric that is a constituent mainly composed of the given thermoplastic resin and that are mainly hydrophobic non-woven fabric is performed. In general, the non-woven fabric has a form of sheet and thus non-woven fabric fragments that are fragments that are mainly non-woven fabric can be referred to as sheet fragments.

Thus, in the separating process, in addition to separation of sheet fragments that are fragments that are mainly hydrophobic non-woven fabric from the fragments including various constituents like those described above, the sheet fragments are separated also from various foreign materials. Note that the hydrophobic non-woven fabric that is separated and collected is naturally in a form of chips.

In the fragmenting and separating step, it is preferable to fragment non-woven fabric products composed of only polypropylene and polyethylene materials.

Sterilizing Step

A sterilizing step at step S23 will be described. For example, to be accepted as a recycling object on which the recycling process is to be performed, it is necessary to meet the recycling conditions like those illustrated in FIG. 3. Specifically, a material that meets the recycling conditions that "the chlorine content is at or under N21%", "the amount of residual bacteria is at or under N22%", and "ash is at or under N23%" is accepted as a recycling object that is an object on which the recycling process is to be performed and is subjected to the recycling process.

For this reason, at the sterilizing step, the sterilizing process of performing sterilizing (disinfection) cleaning on the collected materials that are separated and collected is performed to obtain a material that meets the above-described recycling conditions. For example, at the sterilizing step, the sterilizing process of performing sterilizing cleaning on the collected materials using a given non-chlorine solution is performed. An ozone solution (for example, ozone water) is taken as the non-chlorine solution. At the sterilizing step, the sterilizing process may be performed using a disinfection agent into which ozone, chlorine dioxide, hypochlorous acid, a hydrogen peroxide solution, peracetic acid, or the like, is mixed or the sterilizing process may be performed using hot water at or above 70 degree Celsius.

About Cleaning and Separating Step

The cleaning and separating step at step S24 will be described. The cleaning and separating step is a step that is added in the pre-processing method PT2 additionally. For example, in chemical recycling, as illustrated in FIG. 3, after a recycling raw material that is a macromolecular polymer is converted into oil and thus is turned into a monomer temporarily, re-polymerization is performed and accordingly a new macromolecular polymer is obtained, and fiberization is performed using the macromolecular polymer. Thus, for example, when conversion into oil is performed with a substance (impurities) that cannot be converted into oil, such as pulp, being mixed, the impurities are mixed into the macromolecular polymer obtained by re-polymerization and, as a result, fiberization cannot be performed properly. For example, when there are a lot of impurities, disconnection tends to occur during fiberization and accordingly fibers in a determined length cannot be obtained. For this reason, in chemical recycling, it is required to put a high-purity macromolecular polymer with fewer impurities in as a recycling raw material.

A step that is added to solve the problem unique to the chemical recycling is the cleaning and separating step at step S24. Specifically, at the cleaning and separating step, a cleaning and separating process of separating (washing off) impurities that are attached to the hydrophobic non-woven fabric by cleaning the non-woven fabric using a given polar solvent is performed. A highly polar solvent, such as water, ethanol, methanol, acetic acid, diethyl ether, or acetone can be taken as the given polar solvent.

The reason of being unable to be converted into oil is that impurities are hydrophilic. Thus, cleaning the hydrophobic non-woven fabric with a highly polar solvent makes it possible to effectively separate the highly-polar hydrophilic impurities (for example, hydrophilic stains and a hydrophilic material) from the low-polar hydrophobic non-woven fabric.

The cleaning and separating step at step S24 thus is a method for, utilizing hydrophobicity of the non-woven fabric, efficiently obtaining a recycling raw material optimum to chemical recycling (specifically, a macromolecular polymer controlled such that impurities are fewer).

Summary

As described above, in the pre-processing method PT2 that is executed as the pre-processing of the recycling process, non-woven fabric products that are used non-woven fabric products mainly composed of the given thermoplastic resin and whose non-woven fabric that is a constituent is hydrophobic are selectively collected (the collecting step), and the collected used non-woven fabric products are fragmented and accordingly the hydrophobic non-woven fabric is separated and collected (the fragmenting and separating step). In the pre-processing method PT2, after the separated and collected hydrophobic non-woven fabric is sterilized and cleaned with the given non-chlorine solution (the sterilizing step), impurities are removed from the hydrophobic non-woven fabric by further cleaning using the given polar solvent (the highly polar solvent) (the cleaning and separating step).

As a result of processing the hydrophobic non-woven fabric by the pre-processing method PT2, as illustrated in FIG. 3, the recycling conditions that "the chlorine content is at or under N21%", "the amount of residual bacteria is at or under N22%", and "ash is at or under N23%" are met so that it can be substantially accepted as being made harmless, and it is possible to obtain a polymeric material with fewer impurities that is suitable for chemical recycling. Thus, the polymeric material is determined as "a processed material" that has been processed such that the material can be subjected to the chemical recycling and is transported to the recycling facility where chemical recycling is performed.

The processed material meets the recycling conditions and thus can be referred to as a material in which certain quality or higher is maintained and obtaining the material at the stage prior to the recycling process by the pre-processing method PT2 makes it possible to effectively inhibit scattering of bacteria in transportation to the recycling facility and conveyance to the next step. In other words, according to the pre-processing method PT2, it is possible to exclude the risk of infection at the stage prior to the recycling process.

For the conceptually-presented values like the chlorine content of N21%, the amount of residual bacteria of N22%", and ash of N23%, optimum real numbers are set in accordance with the situation on the processing site. Specific examples of the recycling conditions are presented below.

For example, as for the specific example of the chlorine content of N21%, setting the concentration of chlorine gas that occurs at or under 1 ppm when the material that is processed by the pre-processing method PT1 is heated to a melting point or higher and is gasified is taken as an example. The value of the concentration of 1 ppm is a value achievable by selecting what to be collected and processing with the non-chlorine solution.

As for the specific example of the amount of residual bacteria of N22%, being lower than a value that is determined by linen standards, being at or under a detection limit as for the amount of residual bacteria of a colon bacterial group and staphylococcus aureus (MRSA), and 10,000 bacteria or less per 100 $cm^2$ as for the amount of remaining other bacteria are taken as an example.

As for the specific example of ash of N23%, ash under 10% (more preferably, under 7% and, more preferably, under 3%) is taken as an example.

Note that the three items of the chlorine content at or under N21%, the amount of residual bacteria at or under N22%, and ash at or under N23% are exemplified; however, the three items need not necessarily be satisfied. For example, in chemical recycling, achieving the chlorine content at or under N21% is the most important and, preferably, the amount of residual bacteria at or under N22% and, more preferably, ash at or under N23% may be achieved, too.

The chlorine content has an effect on apparatuses and devices that are used in the pre-processing step and the recycling step and chemical reactions, the amount of residual bacteria has an effect on safety of operating staffs and on whether acceptance is enabled according to the level of measures against bacteria at various types of process steps and therefore they are thought to have an effect on versatility. Ash has an effect on the properties of the material after recycling and therefore an appropriate value has to be achieved according to the purpose at the sage of the pre-processing step.

3-4. Specific Example of Pre-processing Method PT2

Using FIG. 4, a specific example of the pre-processing method PT1 according to the embodiment will be described. FIG. 2 is a sequence chart illustrating an example of the pre-processing method PT1 according to the embodiment. FIG. 4 illustrates a detailed example of the pre-processing method PT1 described using FIG. 3. FIG. 4 illustrates an example in which the pre-processing method PT1 is performed among a collecting facility CF1, the fragmenting apparatus 10-1, the separating apparatus 10-2, and the sterilizing apparatus 10-3.

In the collecting facility CF1, product collection corresponding to the collecting step among the process steps constituting the pre-processing method PT2 is performed. The fragmenting apparatus 10-1 performs the fragmenting process contained in the fragmenting and separating step among the process steps constituting the pre-processing method PT2. The separating apparatus 10-2 performs the separating process contained in the fragmenting and separating step among the process steps constituting the pre-processing method PT1. The sterilizing apparatus 10-3 performs the sterilizing process corresponding to the sterilizing step among the process steps constituting the pre-processing method PT2. Following the sterilizing process, the sterilizing apparatus 10-3 is also able to perform the cleaning and separating process corresponding to the cleaning and separating step.

Aspects that are common to or similar to those according to the description of FIG. 2 in the description of FIG. 4 will be omitted as appropriate.

In the example in FIG. 4, non-woven fabric products to be processed by the pre-processing method PT2 will be described as masks (mask products) below. Assume that the mask products are products manufactured by the manufacturer M1 and are the mask products PD2 that belong to the brand B1.

The main constituent constituting the mask product PD1 is non-woven fabric. Thus, the mask product PD2 is a mask containing hydrophobic non-woven fabric as a constituent. In other words, the mask product PD2 is a mask containing, as a constituent, non-woven fabric that is mainly composed of a hydrophobic thermoplastic resin and thus presents hydrophobicity. In this respect, for example, the mask product PD2 may be a mask that contains, as a constituent, non-woven fabric mainly composed of a thermoplastic resin whose degree of crystallinity is at or under 75% or whose melting point is at or under 170 degree Celsius and that thus represents hydrophobicity. The mask product PD2 may be a mask that contains, as a constituent, non-woven fabric that contains polypropylene or polyethylene at a percentage of 95% or more (for example, at a percentage by weight of 95% or more) and that thus represents hydrophobicity.

In the state, in the example in FIG. 4, first of all, the collecting facility CF1 that is a supermarket collects masks that are mainly composed of the given thermoplastic resin and whose non-woven fabric that is a constituent is hydrophobic, that is, used mask products PD2 selectively from consumers (step S401). The collecting method described with respect to step S201 may be employed to collect the used mask products PD2.

The used mask products PD2 that are collected in the colleting facility CF1 from consumers, for example, are transported by a given carrier to the pre-processing section 10 (step S402). When the used mask products PD2 arrive, an operating staff in the pre-processing section 10 works to put the used mask products PD2 in the fragmenting apparatus 10-1 while reducing the risk of infection and ensuring safety (for example, prevention of scattering and sterilization of droplets).

The fragmenting apparatus 10-1 performs the fragmenting process of fragmenting the used mask products PD2 finely by crushing (step S403). For example, the fragmenting apparatus 10-1 may crush the used mask products PD2 in an underwater environment or an exhaust environment. For example, the fragmenting apparatus 10-1 crushes the used mask products PD2 while performing sterilization in a given disinfection solution environment or a given exhaust environment. For example, the fragmenting apparatus 10-1 has a tank and the used mask products PD1 are put in the tank with a given polar solvent (for example, a highly polar solvent) therein. The given polar solvent may be a highly polar solvent, such as water, ethanol, methanol, acetic acid, diethyl ether, or acetone.

In the state, the fragmenting apparatus 10-1 pours the used mask products PD1 together with the given polar solvent to a coaxial crusher from a port with which the tank is provided and accordingly crushes the used mask products PD2 while performing sterilization. Note that the fragmenting apparatus 10-1 may perform crushing while performing sterilization using an aqueous solution into which a disinfection agent is mixed or an acid aqueous solution as the given polar solvent.

At step S403, the fragmenting apparatus 10-1 fragments the used mask products PD2 such that the size after fragmentation is within a given range. For example, the fragmenting apparatus 10-1 crushes the used masks PD2 by operating the coaxial crusher that is adjusted such that the size of a mask fragment obtained by crushing the used mask products PD2 is within a range of 1 to 50 mm.

The mask fragments that are fragments obtained by the fragmenting process are put in the separating apparatus 10-2 (step S404).

Once the mask fragments are put in, the separating apparatus 10-2 performs the separating process of separating and collecting, from the mask fragments that are put in, mask fragments (that is, sheet fragments) that are the non-woven fabric mainly composed of the given thermoplastic resin and that are mainly hydrophobic non-woven fabric (step S405). For example, the separating apparatus 10-2 separates sheet fragments (non-woven fabric fragments) that are the mask fragments that are mainly the hydrophobic non-woven fabric from the mask fragments containing the various constituents composing the mask product PD2.

The collected materials that are the sheet fragments that are separated and collected by the separating apparatus 10-2 are conveyed to the sterilizing apparatus 10-3 (step S406). For example, the operating staff in the pre-processing section 10 conveys the collected materials to the sterilizing apparatus 10-3 and puts the conveyed collected material in the sterilizing apparatus 10-3. The operating staff works while reducing the risk of infection and ensuring safety (for example, prevention of scattering and sterilization of droplets).

Once the collected materials derived from hydrophobic non-woven fabric are put in, the sterilizing apparatus 10-3 performs the sterilizing process of sterilizing and cleaning the collected materials such that materials that meet the recycling conditions are obtained (step S407). For example, the sterilizing apparatus 10-3 sterilizes and cleans the hydrophobic non-woven fabric with the given non-chlorine solution. For example, the sterilizing apparatus 10-3 sterilizes and cleans the collected materials using an oxidant non-chlorine solution (for example, an ozone solution, such as ozone water) as the given non-chlorine solution.

For example, the sterilizing apparatus 10-3 is the apparatus described with respect to step S207 and sterilizes and cleans the collected materials by spraying the given non-chlorine solution while stirring the collected materials in a gas phase. The sterilizing process sterilizes and cleans the collected materials while applying physical damages to the collected materials and therefore makes it possible to realize an increase in efficiency of sterilization, shortening of the time taken by the sterilizing process, and a reduction in the amount of the used solution.

Only sterilizing and cleaning at step S407 sometimes do not necessarily remove impurities (the substance unable to be converted into oil) having an adverse effect on the chemical recycling from the collected materials. Accordingly, the sterilizing apparatus 10-3 performs the cleaning and separating process of separating impurities that are attached to the collected materials by cleaning the collected materials with the given polar solvent (step S408).

For example, the sterilizing apparatus 10-3 is able to perform the cleaning and separating process on the collected materials in the cylindrical part that is described with respect to step S207 in FIG. 2.

For example, with the blades being rotated, by spraying the highly polar solvent at a high pressure to the collected materials that are put in the cylindrical part, the sterilizing apparatus 10-3 separates the hydrophilic impurities (for example, hydrophilic stains and a hydrophilic material) that are also highly polar from the low-polar collected materials. More specifically, while rotating the blades to send the collected materials from the port of the cylindrical part to the exit, the sterilizing apparatus 10-3 performs the cleaning and separating by spraying the highly polar solvent at a high pressure to the collected materials.

According to the cleaning and separating process, the cleaning and separating are performed using the polar solvent while the collected materials derived from hydrophobic non-woven fabric are being applied with physical damages, which makes it possible to realize effective removal of impurities. The cleaning and separating process makes, as a result, the collected materials derived from hydrophobic non-woven fabric meet the recycling conditions and become a polymeric material with fewer impurities that is suitable for the chemical recycling.

The pre-processing method PT2 described as a process dedicated to the chemical recycling may be performed also in the pre-processing method PT1. Specifically, the cleaning and separating step performed in the pre-processing method PT2 may be incorporated in the process steps in the pre-processing method PT1. More specifically, the cleaning and separating process described with respect to step S408 may be performed, for example, subsequently also after the sterilizing process that is performed at step S207.

When the cleaning and separating process at step S408 ends, the sterilizing apparatus 10-3 performs a desolvation process on the collected materials from which the impurities have been removed, thereby making an adjustment such that the moisture content of the collected materials is at or under a given value (step S409).

The sterilizing apparatus 10-3 performs an evaluating process of evaluating whether the collected materials after desolvation meet the recycling conditions (step S410). Specifically, the sterilizing apparatus 10-3 evaluates whether the collected materials after desolvation meet the recycling conditions that "the chlorine content is at or under N21%", "the amount of residual bacteria is at or under N22%", and "ash is at or under N23% (for example, 3.0%)".

When an evaluation result presenting that any one of the recycling conditions is not met is obtained (NO at step S410), the process from step S407 is repeated until an evaluation result presenting that all the recycling conditions are met is obtained.

When the evaluation result presenting that all the recycling conditions are met is obtained (YES at step S410), the sterilizing apparatus 10-3 moves to an extracting step. For example, when the evaluation result presenting that all the recycling conditions are met is obtained, the sterilizing apparatus 10-3 extracts the hydrophobic non-woven fabric that meets all the recycling conditions as processed materials (the recycling raw material) in which certain quality or higher is ensured such that the materials can be subjected to the recycling process (step S411) .

Note that, at step S410, all the recycling conditions need not necessarily be met and, for example, recycling conditions that should be met may be changed according to the purpose of recycling. The collected processed materials are transported to a given recycling facility where the chemical recycling is performed (step S412) .

The specific example of the pre-processing method PT2 according to the embodiment has been described using FIG. 4. According to the pre-processing method PT2 described above, it is possible to obtain the recycling raw material that is a high-purity macromolecular polymer with fewer impurities and that is neutralized and therefore it is possible to produce a high-quality material as a material of new products or reproduce high-quality non-woven fabric products.

4. Tracking Utilizing Blockchain

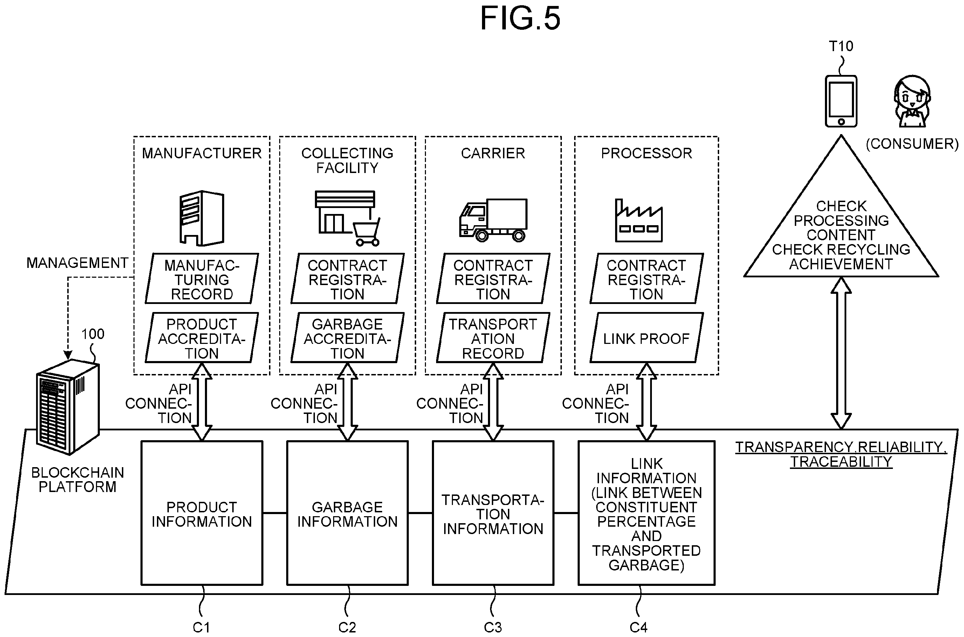

Using FIGS. 1 to 4, the recycling technique containing the pre-processing method according to the embodiment has been described; however, information processing that increases transparency of a supply chain of non-woven fabric products using a blockchain may be realized in the recycling systems illustrated with respect to FIG. 1 or FIG. 3. The supply chain herein denotes, for example, a flow from manufacturing of non-woven fabric products to recycling utilizing collection, transportation, and the pre-processing method and consumption. An example of such information processing utilizing a blockchain will be described using FIG. 5. FIG. 5 is a diagram illustrating an example of the blockchain according to the embodiment.

FIG. 5 illustrates an information processing device 100 as an example of the information processing device that manages a blockchain. The information processing device 100 is, for example, a server device. In the example in FIG. 5, the information processing device 100 is managed by a manufacturer M1 that manufactures the mask products PD1 and PD2. On the other hand, the information processing device 100 need not belong to any of member organizations (in the example in FIG. 5, the manufacturer M1, a collecting facility, a carrier, and a processor) joining a blockchain.

For example, the information processing device 100 may regard one content as one block and connect the dependencies thereof like a chain, thereby making it possible to sense manipulation on the content. For example, as for information ensuring the truth, the information processing device 100 may verify the truth in association with the blockchain and then perform information preservation. For example, as for information on falsification, the information processing device 100 may verify the falsification in association with the blockchain and then perform information preservation.

For example, the information processing device 100 may use various conventional techniques on hashes, such as various hash functions, etc. For example, the information processing device 100 may use techniques of SHA (Secure Hash Algorithm), such as SHA-256 and SHA-512. For example, the information processing device 100 may use a technique of RIPEMD-160. The information processing device 100 may use not only hashes but also any technique as long as it is possible to sense that no falsification (change) is made in the content. For example, the information processing device 100 may use various conventional techniques on elliptic curve cryptography, such as an elliptic curve DSA (Digital Signature Algorism).

In the example in FIG. 5, using a platform in which an API connection with the information processing device 100 is made, the manufacturer M1 registers product information C1 (an example of content) containing a manufacturing record on the mask products PD1 (or the mask products PD2) and product accreditation that is information on the mask product PD1 that is accredited as a product. In the example in FIG. 5, using the platform in which an API connection with the information processing device 100 is made, the collecting facility registers garbage information C2 (an example of content) containing a contract that proves that it is an official place of collection where the mask products PD1 are collected and garbage accreditation indicating that the collected mask products PD1 are accredited as garbage.

In the example in FIG. 5, using the platform in which an API connection with the information processing device 100 is made, the carrier registers transportation information C3 (an example of content) containing a contract that proves that it is an official organization that transports the mask products PD1 that are collected in the collecting facility and a transportation record on transportation of the mask products PD1 that are collected in the collecting facility.

In the example in FIG. 5, using the platform in which an API connection with the information processing device 100 is made, the processor registers link information C4 (an example of content) containing a contract that proves that it is an official organization that performs the recycling process on the mask products PD1 that are transported and a link proof that proves the link between the content percentage of thermoplastic resin in the mask products PD1 and a recycling result of recycling the mask products PD1 that are transported.

According to the example in FIG. 5, the information processing device 100 associates the product information C1, the garbage information C2, the transportation information C3, and the link information C4 using the block chain.

According to the example in FIG. 5, a consumer is able to access the information processing device 100 via the platform using a terminal device T10 (for example, a smartphone) of the consumer and check the content of processing and achievement of recycling.

As described, because the information processing device 100 is able to create an audit certificate that is transparent and unchangeable, a consumer is able to track the raw material of the mask product PD1 accurately. For example, the records on the blockchain are arranged in date order in a form that cannot be falsified and therefore a consumer is able to track the process from shipment of waste to recycling.

According to the information processing device 100, because the member organizations are able to view the same information, transparency can be ensured. According to the information processing device 100, the member organizations can check information mutually and therefore reliability can be ensured (in other words, falsification of information and registration of false information can be inhibited).

Although not illustrated in FIG. 5, tags, such as IoT sensors or QR codes, may be added to products in a manufacturing premise and, in this case, the information processing device 100 may, for example, record geographical information that is collected in the tags.

5. About Evaluation Method

In the above-described embodiment, it has been described that the recycling conditions, such as the chlorine content at or under N11% (N21%), the amount of residual bacteria at or under N12% (N22%), and ash at or under N13% (N23%), have to be achieved by the pre-processing step. Thus, for example, step S209 in FIG. 2 presents that the evaluating process of evaluating whether the collected materials meet the recycling conditions is performed. Step S410 in FIG. 4 presents that the evaluating process of evaluating whether the collected materials meet the recycling conditions is performed, too. An example of the specific method that can be employed in the evaluating process will be described below according to each item (the chlorine content, the amount of residual bacteria, and ash).

5-1. Example of Method of Evaluating Chlorine Content

A chlorine concentration in a gas obtained by heating a material to be measured (a sample sampled from the collected materials in the examples of FIG. 2 and FIG. 4) to a fusing point or higher and gasifying the sample is measured. The result of the measurement is determined as a concentration of chlorine contained in the material (that is, the chlorine concentration). Note that the fusing point can be measured using, for example, a differential scanning calorimeter (DSC).

5-2. Example of Method of Evaluating Amount of Residual Bacteria

For example, evaluations on Escherichia coli and general bacteria can be carried out according to Order No. 1 of Showa 37 by Ministry of Health and Welfare and Ministry of Construction.

5-3. Example of Method of Evaluating Ash

Strongly heat a crucible made of platinum, quartz, or porcelain at 500 to 550 °C for one hour previously and, after radiational cooling, measure a mass W0 thereof precisely. Sample 2 to 4 g of a sample in addition to what is additionally specified, put the sample in the crucible, measure a mass W1 thereof precisely, take the lid of the crucible out or shift the lid if necessary, initially heat the crucible lightly, increase the temperature gradually and heat the crucible strongly at 500 to 550 °C for four hours or more, and ash the sample until no carbide remains. After radiational cooling, measure the mass thereof precisely. Ash residues again and, after radiational cooling, measure the mass thereof precisely and repeat ashing, radiational cooling, and weighting until a constant mass. In this method, when carbide still remains and a constant mass is not reached, add hot water for leaching, perform filtration with a filter paper for quantitative analysis, heat residues strongly together with the filter paper and impurities on the filter paper until the carbide disappears. After adding a colature thereto, evaporate and exsiccate and heat it strongly. After radiational cooling, measure the mass precisely. When the carbide still remains even with this method, add a small amount of ethanol for moisture, crush the carbide with a glass rod, clean the glass rod with a small amount of ethanol and, after evaporating the ethanol carefully, perform an operation like the previous one. Perform radiational cooling in a desiccator with silica gel therein. A mass after being a constant weight is W2. Calculate ash (percentage by mass) from the equation below.

$$ash\ (percentage\ by\ mass) = (W2-W0)/(W1-W0) \times 100$$

6. Others

All or part of the processes that are described as those performed automatically among the above-described processes may be performed manually. All or part of the processes that are described as those performed manually may be performed automatically by known methods. In addition to this, the process procedures, specific names, and information including various types of data and parameters that are presented in the above description and the drawings are changeable freely unless otherwise noted. For example, various types of information illustrated in the drawings are not limited to the information illustrated in the drawings.

Each component of each device illustrated in the drawings is a functional idea and need not necessarily be configured physically as illustrated in the drawings. In other words, specific modes of distribution and integration of each device are not limited to those illustrated in the drawings. All or part of each component can be configured by functional or physical distribution or integration in any unit according to various types of load and usage. Each set of processing described above may be combined as appropriate in a range without inconsistency and be executed.

Reference Signs List

CF1 COLLECTING FACILITY
10 PRE-PROCESSING SECTION
10-1 FRAGMENTING APPARATUS
10-2 SEPARATING APPARATUS
10-3 STERILIZING APPARATUS
100 INFORMATION PROCESSING DEVICE

**Claims**

1. A pre-processing method that is executed as processing prior to a recycling process for recycling infectious waste into a material of new products, the pre-processing method comprising:

   a fragmenting step of fragmenting, as the infectious waste, a used non-woven fabric product mainly composed of a given thermoplastic resin; and
   a processing step of processing a part that is obtained at the fragmenting step using a given non-chlorine solution.

2. The pre-processing method according to claim 1, wherein the fragmenting step includes separating hydrophobic non-woven fabric by fragmenting the non-woven fabric product whose non-woven fabric that is a constituent is hydrophobic, and

the processing step includes processing, using the given non-chlorine solution, the separated hydrophobic non-woven fabric as the part that is obtained at the fragmenting step.

3.  The pre-processing method according to claim 1 or 2, wherein the processing step includes processing the part, which is obtained at the fragmenting step, using an oxidant non-chlorine solution as the given non-chlorine solution.

4.  The pre-processing method according to any one of claims 1 to 3, wherein the processing step further includes a step of processing the part that is obtained at the fragmenting step using a given polar solvent.

5.  A pre-processing method that is executed as processing prior to a recycling process for recycling infectious waste into a material of new products, the pre-processing method comprising:

    a fragmenting step of fragmenting, as the infectious waste, a non-woven fabric product that is a used non-woven fabric product that is mainly composed of a given thermoplastic resin and whose non-woven fabric that is a constituent is hydrophobic; and
    a processing step of processing hydrophobic non-woven fabric among parts that are obtained at the fragmenting step using a given polar solvent.

6.  The pre-processing method according to claim 2 or 5, wherein the hydrophobic non-woven fabric is non-woven fabric made of a hydrophobic thermoplastic resin.

7.  The pre-processing method according to any one of claims 1 to 6, wherein the fragmenting step includes fragmenting, as the given thermoplastic resin, a used non-woven fabric product mainly composed of a thermoplastic resin whose degree of crystallinity is at or under 75% or whose melting point is at or under 170 degree Celsius.

8.  The pre-processing method according to any one of claims 1 to 7, wherein the fragmenting step includes fragmenting, as the used non-woven fabric product mainly composed of the given thermoplastic resin, a non-woven fabric product containing polypropylene or polyethylene at a percentage of 95% or more.

9.  The pre-processing method according to any one of claims 1 to 8, wherein the processing step includes processing the part obtained at the fragmenting step while stirring the part together with a given disinfection solution in a gas phase.

10. The pre-processing method according to any one of claims 1 to 9, further comprising an extracting step of extracting, as a processed material on which the recycling process can be to be performed, a material that is determined as meeting a given condition among materials that are obtained via the processing step.

11. The pre-processing method according to claim 10, wherein the extracting step includes extracting, as the processed material, a material that is determined as meeting a condition that a chloride component that is contained is at or under a given value among the materials obtained via the processing step.

12. The pre-processing method according to claim 10 or 11, wherein the extracting step includes extracting, as the processed material, a material that is determined as meeting a condition that an amount of residual bacteria is at or under a given value among the materials obtained via the processing step.

13. The pre-processing method according to any one of claims 10 to 12, wherein the extracting step includes extracting, as the processed material, a material that is determined as meeting a condition that ash is at or under 3.0% among the materials obtained via the processing step.

14. The pre-processing method according to any one of claims 1 to 13, wherein the fragmenting step includes, as a process of fragmenting the used non-woven fabric product mainly composed of the given thermoplastic resin, crushing the non-woven fabric product under a given environment that makes it possible to prevent bacteria from scattering, and
    the processing step includes processing a part after crushing.

15. The pre-processing method according to claim 14, wherein the fragmenting step includes crushing the used non-woven fabric product under an underwater environment or an exhaust environment.

16. The pre-processing method according to claim 14 or 15, wherein the fragmenting step includes crushing the used non-woven fabric product such that a size after crushing is within a given range.

17. A pre-processing system that performs processing prior to a recycling process for recycling infectious waste into a material of new products, the pre-processing system comprising:

   a fragmenting unit that fragments, as the infectious waste, a used non-woven fabric product mainly composed of a given thermoplastic resin; and
   a processing unit that processes a part that is obtained by the fragmenting unit using a given non-chlorine solution.

18. A pre-processing system that performs processing prior to a recycling process for recycling infectious waste into a material of new products, the pre-processing system comprising:

   a fragmenting unit that fragments, as the infectious waste, a non-woven fabric product that is a used non-woven fabric product that is mainly composed of a given thermoplastic resin and whose non-woven fabric that is a constituent is hydrophobic; and
   a processing unit that processes hydrophobic non-woven fabric among parts that are obtained by the fragmenting unit using a given polar solvent.

# FIG.1

INTO NON-
WOVEN
FABRIC
PRODUCTS

S11
COLLEC-
TING
STEP

S12
FRAGMENTING AND
SEPARATING STEP

S13
STERILIZING
STEP

RECYCLING
PROCESS

（MATERIAL
RECYCLING OR
CHEMICAL
RECYCLING）

FIG.2

# FIG.3

S21 COLLEC-TING STEP

INTO NON-WOVEN FABRIC PRODUCTS

CHEMICAL RECYCLING PROCESS

FIBERIZA-TION

S22 FRAGMENTING AND SEPARATING STEP

CONVERSION INTO OIL

S23 STERILIZING STEP

S24 CLEANING AND SEPARATING STEP

FIG.4

CF1
COLLECTING FACILITY

10-1
FRAGMENTING APPARATUS

10-2
SEPARATING APPARATUS

10-3
STERILIZING APPARATUS

10

S401
SELECTIVELY COLLECT USED MASKS WHOSE NON-WOVEN FABRIC BEING CONSTITUENT IS HYDROPHOBIC

S402
TRANSPORT AND PUT IN USED MASKS

S403
FRAGMENTING PROCESS

S404
PUT MASK FRAGMENTS IN

S405
SEPARATING PROCESS

S406
CONVEY AND PUT IN COLLECTED MATERIALS DERIVED FROM HYDROPHOBIC NON-WOVEN FABRIC

S407
STERILIZING PROCESS

S408
CLEANING AND SEPARATING PROCESS

S409
DESOLVATION PROCESS

S410
DO COLLECTED MATERIALS MEET RECYCLING CONDITIONS?

NO

YES

PRE-PROCESSING SECTION

S411
EXTRACT NON-WOVEN FABRIC MEETING RECYCLING CONDITIONS AS PROCESSED MATERIAL (RECYCLING RAW MATERIAL)

S412
TRANSPORT PROCESSED MATERIAL

(RECYCLING FACILITY)

# FIG.5

EP 4 252 991 A1

## INTERNATIONAL SEARCH REPORT

| International application No. |
|---|
| **PCT/JP2021/046931** |

| A. | CLASSIFICATION OF SUBJECT MATTER |
|---|---|

*B29B 17/02*(2006.01)i; *A61L 2/18*(2006.01)i; *A61L 101/10*(2006.01)n
FI:   B29B17/02 ZAB; A61L2/18 100; A61L101:10

According to International Patent Classification (IPC) or to both national classification and IPC

| B. | FIELDS SEARCHED |
|---|---|

Minimum documentation searched (classification system followed by classification symbols)

B29B17/00-B29B17/04; C08J11/00-C08J11/28; A61L2/18; A61L101/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2019-085447 A (UNI CHARM CORP) 06 June 2019 (2019-06-06) | 1-9, 14-18 |
|  | paragraphs [0025]-[0087], fig. 1-6 |  |
| Y |  | 10-13 |
| X | JP 2020-195994 A (UNI CHARM CORP) 10 December 2020 (2020-12-10) | 1-9, 14-18 |
|  | paragraphs [0025]-[0101], fig. 3 |  |
| Y |  | 10-13 |
| Y | JP 7-304040 A (HITACHI LTD) 21 November 1995 (1995-11-21) | 10-13 |
|  | paragraphs [0011]-[0028], fig. 1-2 |  |
| A | JP 2002-273731 A (UNIV FUKUOKA) 25 September 2002 (2002-09-25) | 1-18 |
|  | entire text, all drawings |  |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date |  |  |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means |  |  |
| "P" | document published prior to the international filing date but later than the priority date claimed | "&" | document member of the same patent family |

| Date of the actual completion of the international search | Date of mailing of the international search report |
|---|---|
| **27 January 2022** | **08 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** |  |
|  | Telephone No. |

Form PCT/ISA/210 (second sheet) (January 2015)

**INTERNATIONAL SEARCH REPORT**
Information on patent family members

International application No.

**PCT/JP2021/046931**

| Patent document cited in search report | | | Publication date (day/month/year) | Patent family member(s) | | | Publication date (day/month/year) |
|---|---|---|---|---|---|---|---|
| JP | 2019-085447 | A | 06 June 2019 | WO | 2019/ 087489 | A1 | |
| | | | | TW | 201923195 | A | |
| | | | | CN | 111278901 | A | |
| | | | | KR | 10-2020-0079257 | A | |
| JP | 2020-195994 | A | 10 December 2020 | JP | 2021-637 | A | |
| | | | | JP | 6771633 | B1 | |
| | | | | WO | 2020/241025 | A1 | |
| JP | 7-304040 | A | 21 November 1995 | (Family: none) | | | |
| JP | 2002-273731 | A | 25 September 2002 | (Family: none) | | | |

Form PCT/ISA/210 (patent family annex) (January 2015)

**EP 4 252 991 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006289154 A
- WO 2014357919 A